# EUROPEAN PATENT SPECIFICATION

(11) **EP 0 363 284 B1**
(45) Date of publication and mention of the grant of the patent: **14.05.1997**
(21) Application number: 89402762.2
(22) Date of filing: 06.10.1989
(51) Int. Cl.: C07K 5/02, C07K 5/06, C07K 5/08, C07K 5/10, C07C 271/10, A61K 38/55

(54) **Use of peptidase inhibitors for the preparation of medicaments useful for the treatment of strokes**
Verwendung von Peptidase-Hemmer zur Herstellung von Medikamenten für die Behandlung von Schlaganfällen
Utilisation des inhibiteurs de peptidase pour la préparation de médicaments utiles dans le traitement de l'apoplexie

(30) Priority: 07.10.1988 US 254762
(43) Date of publication of application: 11.04.1990
(73) Proprietor: MERRELL PHARMACEUTICALS INC., Cincinnati, Ohio 45215-6300 (US)
(72) Inventor: Bey, Philippe, Cincinnati Ohio 45242 (US); Angelastro, Michael, Loveland Ohio 45140 (US); Mehdi, Shujaath, Cincinnati Ohio 45213 (US)
(74) Representative: Gillard, Marie-Louise

(56) References cited:
- EP-A- 0 124 317
- EP-A- 0 126 009
- EP-A- 0 128 762
- EP-A- 0 133 225
- EP-A- 0 189 203
- EP-A- 0 195 212
- EP-A- 0 264 106
- EP-A- 0 266 950
- EP-A- 0 281 316
- EP-A- 0 296 581
- EP-A- 0 335 572
- WO-A-84/00365
- WO-A-84/04301
- AU-B- 19 392
- DE-A- 3 000 225
- FR-A- 2 490 632
- CHEMICAL ABSTRACTS, vol. 105, no. 3, 21st July 1986, page 676, abstract no. 24605p, Columbus, Ohio, US; J.A. FEHRENTS et al.: "Synthesis of aldehydic peptides inhibiting renin", & INT. J. PEPT. PROTEIN RES. 1985, 26(3), 236-41
- CHEMICAL ABSTRACTS, vol. 104, no. 15, 14th April 1986, page 312, abstract no. 125573m, Columbus, Ohio, US; G.C. SZABO et al.: "Specific inhibition of human granulocyte elastase with peptide aldehydes", & FEBS LETT. 1986, 195(1-2), 265-8
- CHEMICAL ABSTRACTS, vol. 101, no. 21, 19th November 1984, page 818, abstract no. 192438x, Columbus, Ohio, US; I.J. GALPIN et al.: "Synthetic analogs of the proteinase inhibitor: chymostatin", INT. J. PEPT. PROTEIN RES. 1984, 23(5), 477-86
- CHEMICAL ABSTRACTS, vol. 100, no. 25, 18th June 1984, page 227, abstract no. 205401m, Columbus, Ohio, US; K.A. KOZLOWSKI et al.: "Tumor cell proteinase visualization and quantification using a fluorescent transition-state analog probe", & PROC. NATL. ACAD. SCI. U.S.A. 1984, 81(4), 1135-9
- CHEMICAL ABSTRACTS, vol. 100, no. 23, 4th June 1984, page 250, abstract no.
- 187896v, Columbus, Ohio, US; J.A. FEHRENTZ et al.: "Aldehydic peptides inhibiting renin", & FEBS LETT. 1984, 167(2), 273-6
- CHEMICAL ABSTRACTS, vol. 100, no. 15, 9th April 1984, page 37, abstract no. 114752k, Columbus, Ohio, US; D. BAGDY et al.: "In vitro and ex vivo effect of tripeptide aldehydes on blood coagulation", & KISERL. ORVOSTUD. 1983, 35(6), 650-61
- CHEMICAL ABSTRACTS, vol. 100, no. 3, 16th January 1984, page 242, abstract no. 19717u, Columbus, Ohio, US; I.J. GALPIN et al.: "Analogs of chymostatin", & PEPT. PROC. EUR. PEPT. SYMP., 17th 1982, 649-52
- CHEMICAL ABSTRACTS, vol. 97, no. 3, 19th July 1982, page 339, abstract no. 19651n, Columbus, Ohio, US; S. BAJUSZ et al.: "Structure-activity relationships among the tripeptide aldehyde inhibitors of plasmin and thrombin", & PEPT. SYNTH., STRUCT., FUNCT., PROC. AM. PEPT. SYMP., 7th 1981, 417-20
- CHEMICAL ABSTRACTS, vol. 90, no. 13, 26th March 1979, pages 188-189, abstract
- no. 99132n, Columbus, Ohio, US; S. BAJUSZ et al.: "Inhibition of thrombin and trypsin by tripeptide aldehydes", & INT. J. PROTEIN RES. 1978, 12(4), 217-21
- CHEMICAL ABSTRACTS, vol. 87, no. 25, 19th December 1977, page 278, abstract no. 196211h, Columbus, Ohio, US; R.C. THOMPSON et al.: "Peptide aldehydes: potent inhibitors of serine and cysteine proteases", & METHODS ENZYMOL. 1977, 46, (AFFINITY LABELING), 220-5
- CHEMICAL ABSTRACTS, vol. 86, no. 1, 3rd January 1977, page 511, abstract no. 5859x, Columbus, Ohio, US; & HU- A-12 214 (GYOGYSZERKUTATO INTEZET) 28-09-1976
- CHEMICAL ABSTRACTS, vol. 85, no. 15, 11th October 1976, page 235, abstract no. 105947x, Columbus, Ohio, US; S. BAJUSZ et al.: "Peptide aldehyde inhibitors of the fibrinogen-thrombin reaction", & PEPT.: CHEM., STRUCT. BIOL., PROC. AM. PEPT. SYMP., 4th 1975, 603-8
- CHEMICAL ABSTRACTS, vol. 80, no. 25, 24th June 1974, page 481, abstract no. 146554g, Columbus, Ohio, US; &
- JP-A-73 91 028 (SANKYO CO., LTD) 27-11-1973
- BIOCHEMISTRY, vol. 27, no. 17, 23rd August 1988, pages 6568-6573, American Chemical Society; R.A. SMITH et al.: "Inhibition of cathepsin B by peptidyl aldehydes and ketones: Slow-binding behavior of a trifluoromethyl ketone"
- BIOCHEMISTRY, vol. 25, no. 5, 11th March 1986, pages 1072-1078, American Chemical Society; D. GROBELNY et al.: "Inhibition of angiotensin converting enzyme by aldehyde and ketone substrate analogues"
- BIOCHEMISTRY, vol. 18, no. 8, 17th April 1979, pages 1552-1558, American Chemical Society; R.C. THOMPSON et al.: "Reaction of peptide aldehydes with serine protease. Implications for the entropy changes associated with enzymatic catalysis"
- PEPTIDES, STRUCTURE & FUNCTION - PROCEEDINGS OF THE AMERICAN PEPTIDE SYMPOSIUM, 1985, pages 799-802, Pierce Chemical Co., Rockford, US; I.J. GALPIN et al.: "A new approach to the synthesis of peptide aldehyde inhibitors"
- BIOCHEMICAL AND BIOPHYSICAL RESEARCH COMMUNICATIONS, vol. 157, no. 3, 30th
- December 1988, pages 1117-1123, Academic Press, Duluth, MN, US; S. MEHDI et al. : "Inhibition of the proteolysis of rat erythrocyte membrane proteins by a synthetic inhibitor of calpain"
- PEPTIDES, STRUCTURE & FUNCTION (C.M. DEBER et al.) - PROCEEDINGS OF THE AMERICAN PEPTIDE SYMPOSIUM, 1985, pages 819-822, Pierce Chemical Co., Rockford, US; H. HORI et al.: "Inhibition of human leukocyte elastase, porcine pancreatic elastase and cathepsin G by peptide ketones"
- CHEMICAL ABSTRACTS, vol. 107, no. 25, 21st December 1987, page 21, abstract no. 228458u, Columbus, Ohio, US; J.L. KRAUS et al.: "Effect of new biologically active polypeptides on dihexadecyl phosphate vesicles", & PHARMACOL. RES. COMMUN. 1987, 19(7), 469-77
- TETRAHEDRON LETTERS, vol. 29, no. 28, 1988, pages 3433-3436, Pergamon Press, Oxford, GB; J.P. BURKHART et al.: "Oxidation of alpha-hydroxy esters to alpha- keto esters using the dess-martin periodinane reagent"
- THE JOURNAL OF ORGANIC CHEMISTRY, vol. 54, no. 16, 4th August 1989, pages 3913-
- 3916, American Chemical Society; M.R. ANGELASTRO et al.: "An efficient synthesis of novel alpha-diketone and alpha-keto ester derivatives of N- protected amino acids and peptides"
- BIOCHEMICAL AND BIOPHYSICAL RESEARCH COMMUNICATIONS, vol. 166, no. 2, 30th January 1990, pages 595-600, Academic Press, Inc., San Diego, US; S. MEHDI et al.: "The inhibition of human neutrophil elastase and cathepsin G by peptidyl 1,2-dicarbonyl derivatives"

## Description

This invention relates to protease enzyme inhibitors useful for a variety of physiological end-use applications.

In its broad aspects, this invention relates to analogs of peptidase substrates in which the nitrogen atom of the scissile amide group of the substrate peptide has been replaced by H or a substituted carbonyl moiety. These analogs of the peptidase substrates provide specific enzyme inhibitors for a variety of proteases, the inhibition of which will have useful physiological consequences in a variety of disease states.

In its more specific aspects, this invention relates to derivatives of certain peptidase substrates which are useful in inhibiting serine-, thio-, carboxylic acid- and metallo-dependent protease enzymes, the inhibition of which will have useful physiological consequences in a variety of disease states.

Still more specifically, this invention relates to derivatives of peptidase substrates which fall within the following generic groupings characterized according to their active site dependencies. Such generic groupings are:
I. Serine Dependent Enzymes: These include such enzymes as Elastase (human leukocyte), Cathepsin G, Thrombin, Plasmin, C-1 Esterase, C-3 Convertase, Urokinase, Plasminogen Activator, Acrosin, β-Lactamase, D-Alanine-D-Alanine Carboxypeptidase, Chymotrypsin, Trypsin and Kallikreins.
II. Thiol Dependent Enzymes: Cathepsin B and Calpain
III.Carboxylic Acid Dependent Enzymes: These include such specific enzymes as Cathepsin D.
IV. Metallo Dependent Enzymes: These include Angiotensin Converting Enzymes, Enkephalinase, Pseudomonas Elastase, Leucine Aminopeptidase and HIV.

The contemplated peptidase inhibitors of the foregoing enzymes are selected from the generic formula

R₁NHCHR₂C(O)X

the hydrates, isosteres or the pharmaceutically acceptable salts thereof, wherein
- X: is H or C(O)R₃,
- R₃: is H, methyl, ethyl, OH, methoxy or ethoxy,
- R₁: is H, a protecting group, an α-amino acid or a peptide having 2 to 4 α-amino acids, optionally bearing a protecting group,
- R₂: is a residue of an α-amino acid responsible for directing the inhibitor to the active site of the enzyme or is -A-SiR₇R₈R₉, C₁₋₁₀ alkyl, aralkyl or aryl, with A being a C₁₋₆ alkylene, and each of R₇, R₈ and R₉ being C₁₋₁₀ alkyl, benzyl or phenethyl.

Unless otherwise stated the α-amino acids of the foregoing peptidase substrates are preferably in their L-configuration. A compound of this invention may be in free form, e.g. amphoteric form, or a salt form, e.g., acid addition or anionic salt. A compound may be converted into its salt or base form in an art-known manner, one from another. Preferred salts are trifluoroacetate, hydrochloride, sodium, potassium or ammonium salts, although the scope of salts embraced herein is not limited thereto, the scope being extended to include all of the salts known to be used in the art of peptide chemistry.

As used herein the term "C₁₋₁₀ alkyl" includes the straight, branched-chain and cyclized manifestations thereof, particularly such moieties as methyl, ethyl, n-butyl, t-butyl, cyclopropyl, n-propyl, pentyl, cyclopentyl, n-hexyl, n-nonyl, decyl, cyclohexyl and cyclohexylmethyl. The term "aralkyl" includes those aryl moieties attached to a C₁₋₄ alkylene. The term "aryl" within the definitions of R₂ and R₃ includes both carbocyclic and heterocyclic moieties. Preferred aralkyl and aryl moieties are phenyl, benzyl, naphthylmethyl, phenethyl, 2-pyridylmethyl, indolyl, pyridyl, indazolyl, furyl and thienyl are preferred. Other carbocyclics are such fused aryl moieties as pentalenyl, indenyl, naphthalenyl, naphthylmethyl, azulenyl, heptalenyl, acenaphthylenyl, fluorenyl, phenalenyl, phenanthrenyl, anthracenyl, acephenanthrylenyl, aceanthrylenyl, triphenylenyl, pyrenyl, chrysenyl and naphthacenyl. In the term "-A-SiR₇R₈R₉" the alkylene moiety (i.e. "A") is a straight or branched-chain alkylene moiety separating the "SiR₇R₈R₉" moiety from the carbon atom to which the "-A-SiR₇R₈R₉" radical is attached. Of the R₇, R₈ and R₉ radicals attached to the silicone atom it is preferred that two or three of these radicals be a C₁₋₁₀ lower alkyl radical (preferably methyl or ethyl) and that when one of them contains an aryl radical it is preferred that that radical be a benzyl or phenethyl radical. It is preferred that the alkylene moiety be methylene. Preferred moieties are trimethylsilyl methyl, triethylsilylmethyl, benzyldiethylsilylmethyl, benzyldimethylsilylmethyl, benzylethylmethylsilylmethyl, and the like.

Before further defining and/or illustrating the scope of the peptidase substrate inhibitors embraced by Formula I, it may be convenient to state some of the more basic concepts related to peptides. For example, except for proline, all of the α-amino acids found in proteins have, as a common denominator, a free carboxyl group and a free unsubstituted amino group on the α-carbon atom (in proline, since proline's α-amino group is substituted it is really an α-imino acid, but for convenience, it will also be spoken of as an α-amino group). Additionally, each α-amino acid has a characteristic "R-group", the R-group being the side-chain, or residue, attached to the α-carbon atom of the α-amino acid. For example, the R-group residue for glycine is hydrogen, for alanine it is methyl, for valine it is isopropyl. (Thus, throughout this specification the R₂ moiety is the residue R-group for each indicated α-amino acid). For the specific R-groups - or side chains - of the α-amino acids reference to A.L. Lehninger's text on Biochemistry (see particularly Chapter 4) would be helpful.

As a further convenience for defining the scope of the compounds embraced by the generic concept of Formula 1, as well as the sub-generic concepts relating to each of the individual enzymes involved in this invention, various α-amino acids have been classified into a variety of groups which impart similar functional characteristics for each of the specific enzymes to be inhibited by the peptidase substrates of Formula 1. These groups are set forth in Table II and the recognized abbreviations for the α-amino acid blocks are set forth in Table I.

**TABLE I**

| AMINO ACID | SYMBOL |
|---|---|
| Alanine | Ala |
| Arginine | Arg |
| Aspargine | Asn |
| Aspartic acid | Asp |
| Asn + Asp | Asx |
| Cysteine | Cys |
| Glutamine | Gln |
| Glutamic acid | Glu |
| Gln + Glu | Glx |
| Glycine | Gly |
| Histidine | His |
| Isoleucine | Ile |
| Leucine | Leu |
| Lysine | Lys |
| Methionine | Met |
| Phenylalanine | Phe |
| Proline | Pro |
| Serine | Ser |
| Threonine | Thr |
| Tryptophan | Trp |
| Tyrosine | Tyr |
| Valine | Val |
| Norvaline | n-Val |
| Norleucine | n-Leu |
| 1-Naphthylalanine | Nal(1) |
| 2-Indolinecarboxylic acid | Ind |
| Sarcosin | Sar |

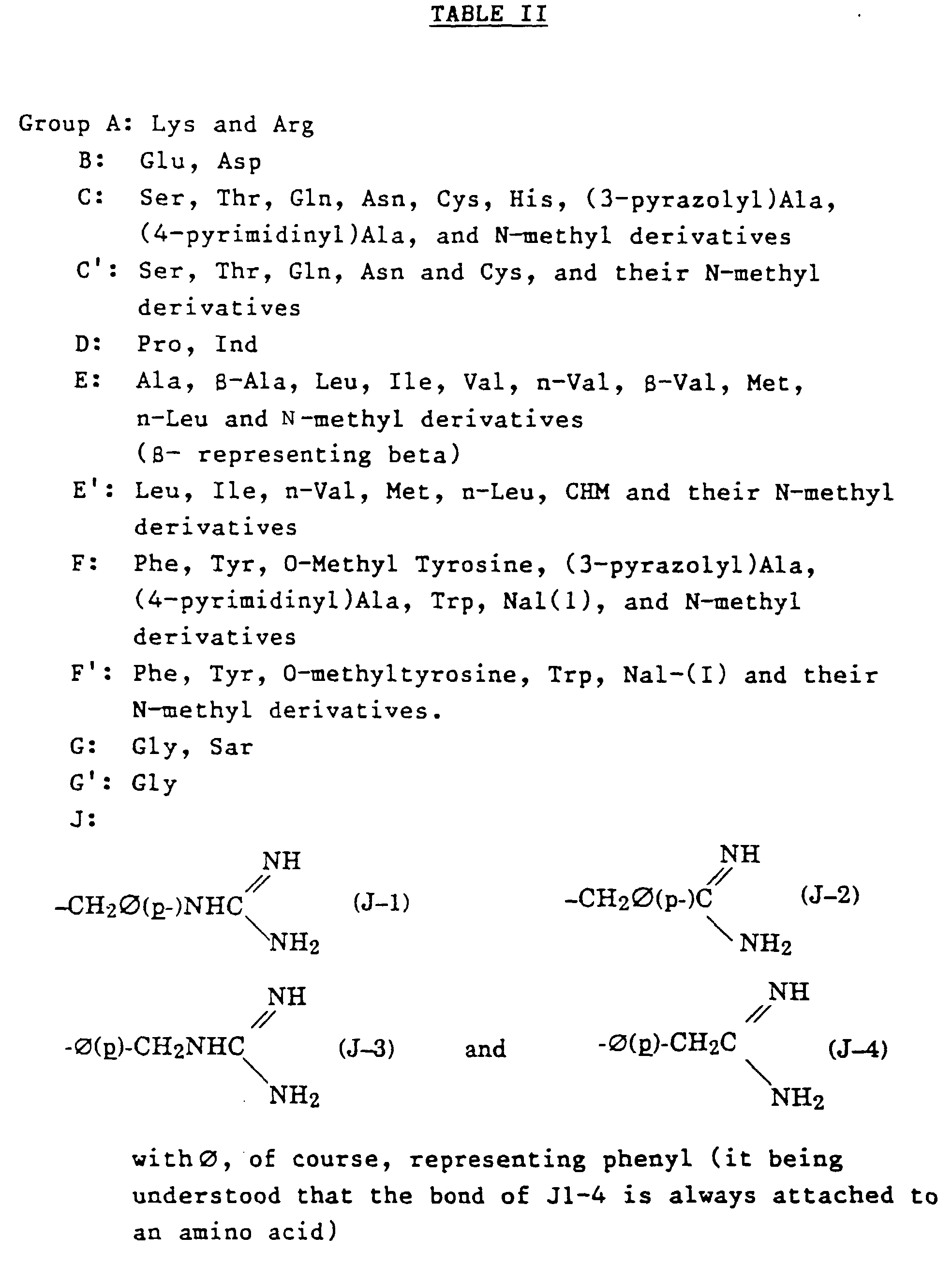

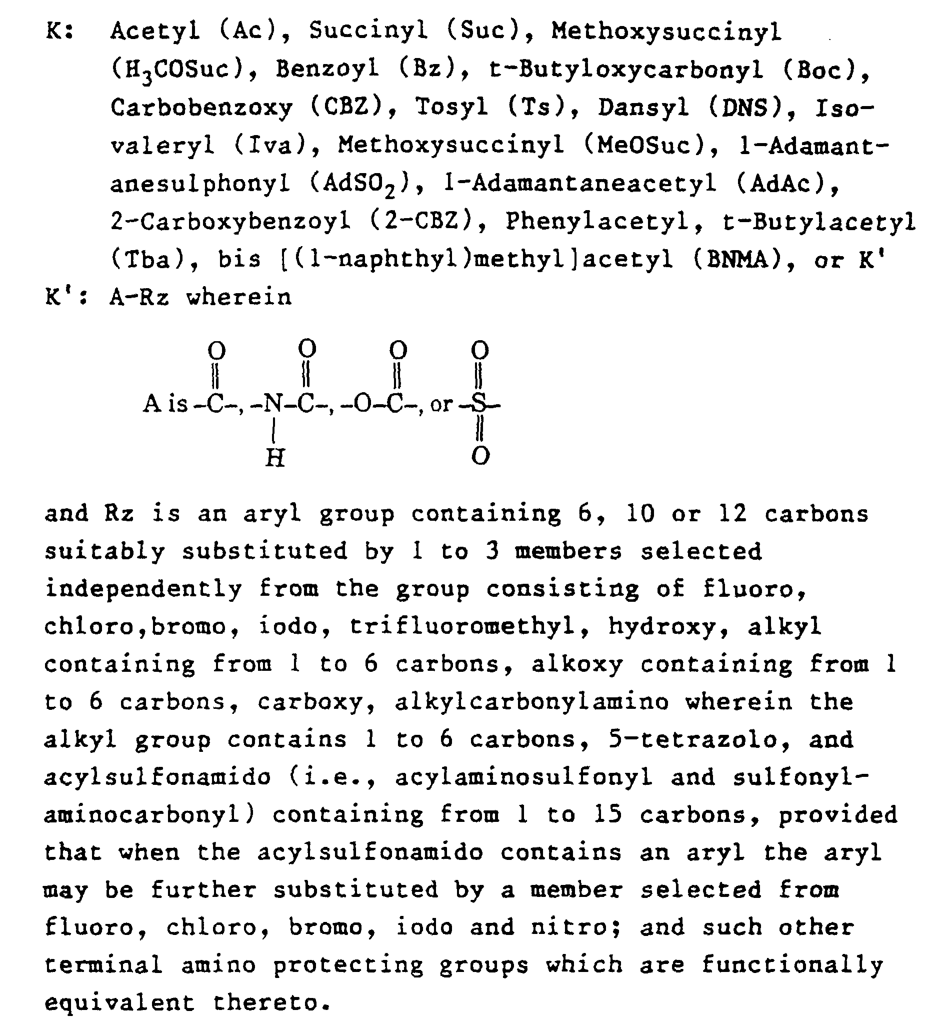

In those instances wherein the normal R-group residue of an α-amino acid contains an -OH radical (e.g. serine, threonine and tyrosine), it is to be understood that such a radical can be derivatized. For example, in each of the foregoing instances the -OH radical can be converted to an ether. When so-converted, such as for example to their methyl ethers, then such radicals will be referred to as 0-methyl Ser, 0-methyl Thr and 0-methyl Tyr, respectively. These methyl ether radicals may also be depicted as 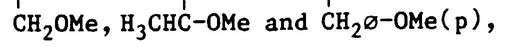 respectively. Similarly, other type derivatives will be analogously represented.

In those instances wherein Group K represents an -A-Rz moiety, it is preferred that A represent -C(=O)- and that Rz represent acylsulfonamido, particularly those wherein the acylsulfonamido contains an aryl moiety (preferably phenyl) substituted by a halogen. The preferred -A-Rz moieties being 4[(4chlorophenyl)sulfonylaminocarbonyl]phenylcarbonyl, 4[(4bromophenyl4[(4bromophenyl)sulfonylaminocarbonyl]phenylcarbonyl and 4[(phenylsulfonylaminocarbonyl]phenylcarbonyl ]phenylcarbonyl (said moieties being abbreviated as 4-Cl-ø-SAC-Bz, 4-Br-ø-SAC-Bz and ø-SAC-Bz, respectively).

Quite obviously the modifications to the scissile amide bond of the peptidase substrates of this invention present certain nomenclature difficulties. In order to maintain a general consistency throughout this application the following explanations are offered to obviate any ambiguities relating to the scope and intent of this invention.

For exemplification, assume R₁ is a dipeptide which contains the two amino acids, Phe and Val, in the P₃ and P₂ positions respectively, the terminal amine of which bears a CBZ moiety of Group K, R₂ is the residue of the P₁-position α-amino acid which in this illustration is Arg. Assume 4 compounds wherein R₃ is either H, methyl, -OH, or OCH₃. In such instances the four different compounds will be written as

CBZ-Phe-Val-Arg[C(O)H],

CBZ-Phe-Val-Arg[C(O)CH₃],

CBZ-Phe-Val-Arg[C(O)-OH],

CBZ-Phe-Val-Arg[C(O)-OCH₃].

The bracketed moiety is used to designate the position wherein the 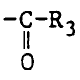 moiety is located. In those instances wherein the α-amino acid located in any of the indicated P-positions contains an N-alkyl (or other) derivative, then for example in the above illustration of CBZ-Phe-Val-Arg[C(O)H], if the nitrogen atom of the P₂ position α-amino acid bore a methyl group, then such compound would be indicated as
CBZ-Phe-N-Me-Val-Arg[C(O)H]. Of course, it is understood that the designations [C(O)H], [C(O)CH₃],[C(O)-OH] and [C(O)-OCH₃] indicate the moieties 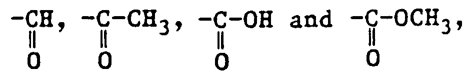 respectively, which are attached to the carbonyl moiety of the P₁ α-amino acid. In the instance wherein X is H then, using the foregoing R₁ and R₂ moieties, the compound would be named CBZ-Phe-Val-Arg-H.

In the light of the foregoing the defined compounds of this invention are compounds of the formulae
and 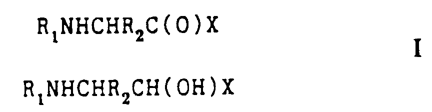 the hydrates, isosteres or the pharmaceutically acceptable salts thereof, wherein
- X: is H or C(O)R₃,
- R₁: is H, a Group K protecting group, an α-amino acid, a peptide comprised of 2 to 4 α-amino acids, an α-amino acid bearing a Group K protecting group, or a peptide comprised of 2 to 4 α-amino acids the terminal α-amino acid of which bears a Group K protecting group,
- R₂: is the residue of an α-amino acid, C₁₋₁₀ alkyl, aralkyl, aryl or -A-SiR₇R₈R₉,
- A: is a C₁₋₆ alkylene, each of
- R₇, R₈ and R₉: are C₁₋₁₀ alkyl, benzyl or phenethyl,
- R₃: is H, methyl, ethyl, OH, methoxy or ethoxy, the said groups of α-amino acids and Group K protecting groups being defined as
A: Lys and Arg
B: Glu, Asp
C: Ser, Thr, Gln, Asn, Cys, His, (3-pyrazolyl)Ala, (4-pyrimidinyl)Ala, and N-methyl derivatives
C': Ser, Thr, Gln, Asn and Cys, and their N-methyl derivatives
D: Pro, Ind
E: Ala, β-Ala, Leu, Ile, Val, n-Val, β-Val, Met, n-Leu and N-methyl derivatives (β- representing beta)
E': Leu, Ile, n-Val, Met, n-Leu, CHM and their N-methyl derivatives
F: Phe, Tyr, 0-Methyl Tyrosine, (3-pyrazolyl)Ala, (4-pyrimidinyl)Ala, Trp, Nal(1), and N-methyl derivatives
F': Phe, Tyr, O-methyltyrosine, Trp, Nal-(I) and their N-methyl derivatives.
G: Gly, Sar
G': Gly
- J:: 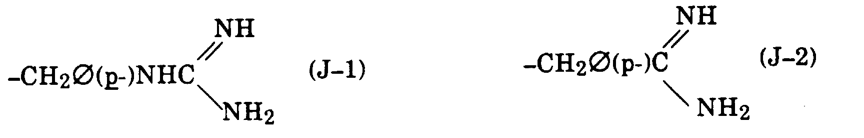 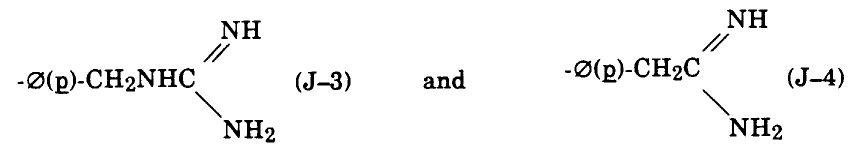
- K:: Acetyl (Ac), Succinyl (Suc), Methoxysuccinyl (H₃COSuc), Benzoyl (Bz), t-Butyloxycarbonyl (Boc), Carbobenzoxy (CBZ), Tosyl (Ts), Dansyl (DNS), Iso-valeryl (Iva), Methoxysuccinyl (MeOSuc), 1-Adamantanesulphonyl (AdSO₂), 1-Adamantaneacetyl (AdAc), 2-Carboxybenzoyl (2-CBZ), Phenylacetyl, t-Butylacetyl (Tba), bis [(1-naphthyl)methyl]acetyl (BNMA), or K'
- K':: is -A-Rz wherein A is 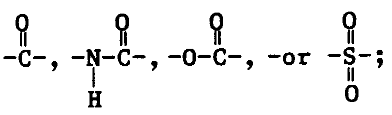 and Rz is an aryl group containing 6, 10 or 12 carbons suitably substituted by 1 to 3 members selected independently from the group consisting of fluoro, chloro,bromo, iodo, trifluoromethyl, hydroxy, alkyl containing from 1 to 6 carbons, alkoxy containing from 1 to 6 carbons, carboxy, alkylcarbonylamino wherein the alkyl group contains 1 to 6 carbons, 5-tetrazolo, and acylsulfonamido containing from 1 to 15 carbons, provided that when the acylsulfonamido contains an aryl the aryl may be further substituted by a member selected from fluoro, chloro, bromo, iodo and nitro.

Compounds of Formula I which are useful as inhibitors of Calpain are compounds of the formula

R₁NHCHR₂C(O)X Iu

the hydrates, isosteres or the pharmaceutically acceptable salts thereof, wherein
- X: is H,
- R₁: is a Group K protecting group, P₂P₃ or P₂P₃P_{g}, P_{g} being a Group K protecting group, preferably the protecting groups are CBZ, Bz or Ac,
P₂ is an α-amino acid of Groups E and F, preferably Ala, Phe or Lys,
P₃ is deleted or is an α-amino acid of Groups B, E or F, preferably it is deleted,
- R₂: is H, the residue of an α-amino acid of Groups E, F and J or-A-Si-R₇R₈R₉, C₁₋₇ alkyl, benzyl phenethyl, or naphthyl, preferably cyclohexylmethyl (CHM), naphthyl (NAP), trimethylsilylmethyl (TMSM), benzyldimethylsilylmethyl (BDMS-M), Lys or Phe.

By their inhibition of Calpain and cathepsin B proteases the compounds of (Iu) will (a) have an effect on cell motility through the extracellular matrix rendering the compounds useful for treating cancer metastases; (b) have the effect on long term changes in regulatory proteins (e.g. down-regulation of protein kinase C and breakdown of the cytoskeleton causing secondary effects on platelet activation such as (for enhancing clot formation) leukocyte degranulation (for treating inflammation and immunological diseases, e.g. arthritis, emphysema, multiple sclerosis, and systemic lupus); (c) have the effect on a general intracellular proteolysis, particular muscle cells, causing secondary effect on ischemia/reperfusion cell death, thereby rendering the compounds useful for treating stroke and heart attacks; and (d) will aid in blocking the lysis of red blood cells rendering the compounds useful in the treatment of conditions associated with excessive hemolysis such as in Sickle cell anemia and in kidney dialysis. It is to be expected that the end-use application dose range will be about 0.01 to 10 mg per kg of body weight per day for an effective therapeutic effect.

The preparation of the compounds of this invention may be effected by standard chemical processes analogously known in the art. The processes are depicted and described as follows: 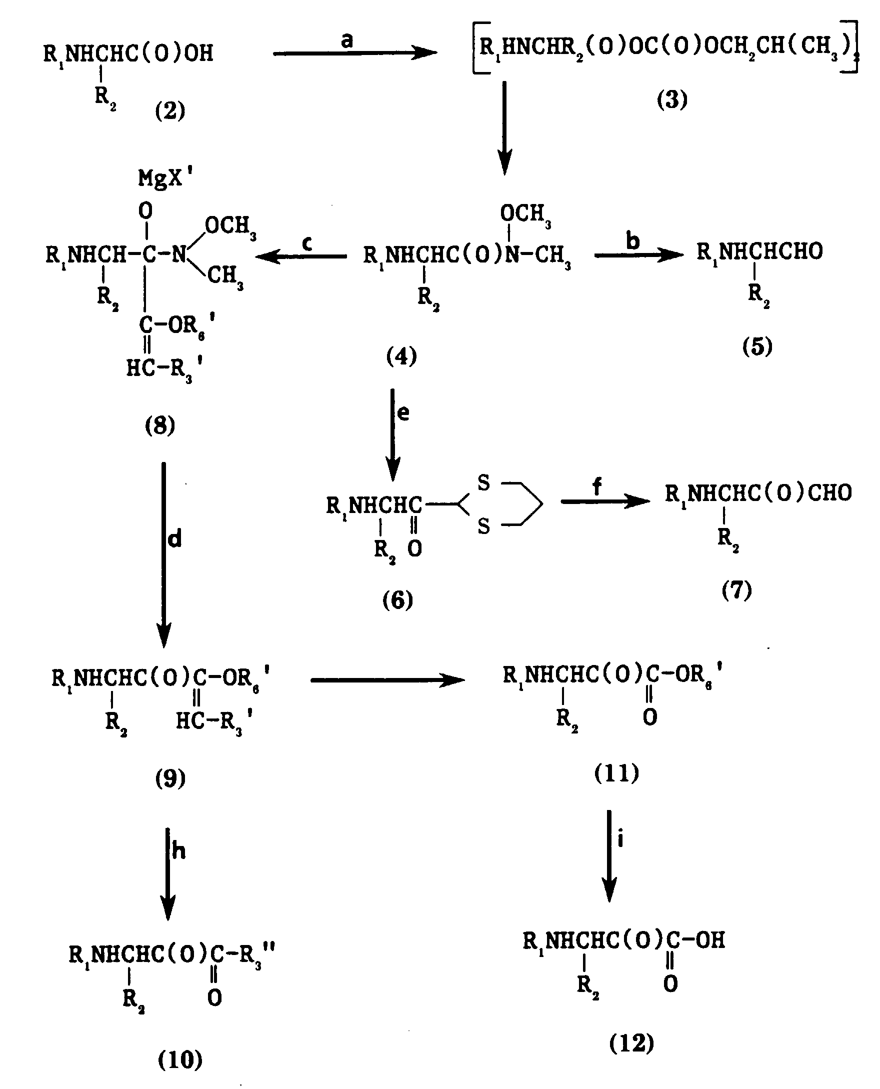 wherein X' is chloro or bromo, R₃' is H or methyl, R₃" is methyl or ethyl, R₆' is methyl or ethyl and R₁ and R₂ are as previously defined.

In effecting the processes of the foregoing reaction scheme, the starting materials (2) are subjected to process step (a) which is initiated by anionizing the starting material with a base, preferably N-methyl morpholine, triethylamine (TEA), diisopropylethylamine (DIEA) or other suitable amines. Preferably the anion is formed using excess quantities of the amine, stirring the mixture at about -15°C to 10°C, preferably 0°C. Addition of an equivalent amount of isobutylchloroformate with cooling at about -20°C forms an *in situ* mixed anhydride (3). (Other equivalently functioning peptide coupling agents, such as diethylcyanophosphonate, DCC, BOP reagents, BOP chloride, may be used in place of isobutylchloroformate.) Addition of molar equivalent amounts of N,O-dimethylhydroxylamine to the activated *in situ* intermediate (3) yields a dimethylhydroxamic acid derivative (i.e. an N-methyl-N-methoxy amide) of Formula (4). This step, as well as reaction steps (b) and (g), are conducted under an inert atmosphere (argon or nitrogen) under anhydrous conditions.

The hydroxamic derivatives (4) may be chemically reduced in step (b) using standard Castro reduction conditions, e.g., lithium aluminum hydride in THF at 0°C or other equivalently functioning reductions, to yield the desired aldehydes (5), or they may be subjected to the reaction conditions of steps (c) and (d) to form compounds 9. Step (c) entails a Grignard reaction using standard reaction conditions such as contacting the reactants together in an inert solvent, preferably tetrahydrofuran, at temperatures of about -20°C to 0°C. The Grignard is freshly prepared from an organo lithium species, e.g., t-butyl lithium added to ethyl vinyl ether which is converted to an ethyl vinyl ether Grignard reagent by reaction with magnesium bromide using standard procedures well known in the art. The hydroxamic derivatives (4) are added to the Grignard reagent to form an *in situ* Grignard complex (8) which, by Step (d), is converted to an α-keto vinyl ether (9), said α-keto vinyl ether being converted by treatment with hydrochloric acid in a dioxane-water mixture or any other inert solvent such as tetrahydrofuran, to the desired diketones of Formula (10).

To obtain the desired α-keto aldehyde of Formula (7), the hydroxamic acid derivatives (4) may be subjected to a nucleophilic attack by 2-metallo-1,3-dithiane according to the techniques of D. Seebach and E.J. Corey [J. Org. Chem., Vol. 40, page 231, (1975)] to form compounds (6). Preferably 2-metallo-1,3-dithiane is formed by addition of a slight excess (5%) of n-butyllithium to a solution of 1,3-dithiane in tetrahydrofuran cooled at -40°C. To this solution is added ½ equivalent of derivatives (4) in an inert solvent and the mixture is stirred at a temperature of about -20°C to 20°C for 1 to 24 hours. The thioketal derivatives (6) may be hydrolyzed to the desired ketoaldehyde derivatives (7) by following standard procedures [J. Org. Chem., 36, 3553 (1971)] such as the use of Lewis acids, HgCl₂, or BF₃ etherate, in the presence of an insoluble base, HgO or CO₃CO₂ in aqueous polar solvents, or the use of an oxidative agent, i.e. N-halosuccinimide in aqueous acetonitrile.

To obtain the desired α-keto esters, the ethyl vinyl ethers of Formula (9) are subjected to an ozonolysis (g) which entails treatment with ozone in methylene chloride or other inert solvents at -78°C under an inert atmosphere (N₂ or Ar) to form an *in situ* ozonide which is converted by treatment with dimethylsulfide to form the desired α-keto esters of Formula (11). These compounds (11) may then be subjected to an acid or base catalyzed hydrolysis (preferably LiOH) to produce compounds of Formula (12).

Of course, in those instances wherein it is more convenient for synthesis the compounds of Formulae VII and XI wherein R₁ is a protecting group (preferably BOC) may be prepared by analogous chemical processes and then such compounds would be subjected to solid-phase sequential and block phase synthetic techniques in order to prepare compounds having the requisite R₁ moiety.

The solid phase sequential procedure can be performed using established automated methods such as by use of an automated peptide synthesizer. In this procedure an amino protected amino acid is bound to a resin support at the carboxy terminal end, the amino acid is deprotected at the amino position at which a peptide linkage is desired, the amino group neutralized with a base and the next amino protected amino acid in the desired sequence is coupled in a peptide linkage. The deprotection, neutralization and coupling steps are repeated until the desired polypeptide is synthesized. The compounds of the present invention are thus synthesized from their carboxy terminal end to their amino terminal end. The amino protected amino acid can be a conventional amino acid, a derivative or isomer thereof, or a spacer group. The resin support employed can be any suitable resin conventionally employed in the art for the solid phase preparation of polypeptides. The preferred resin is polystyrene which has been cross-linked with from about 0.5 to about 3% divinyl benzene, which has been either benzhydrylamidated, chloromethylated or hydroxymethylated to provide sites for amide or ester formation with the initially introduced amino protected amino acid.

An example of a hydroxymethyl resin is described by. Bodansky et al. [Chem. Ind. (London) 38, 1597-98 (1966)]. The preparation of chloromethyl and benzhhydrylamine resins are described by Stewart et al. ["Solid Phase Peptide Synthesis", 2nd Edition, Pierce Chemical Co., Rockford, Illinois (1984), Chapter 2, pp. 54-55]. Many of these resins are available commercially. In general, the amino protected amino acid which is desired on the carboxy-terminal end of the peptide is bound to the resin using standard procedures and practices as are well known and appreciated in the art. For example, the amino protected amino acid can be bound to the resin by the procedure of Gisin [Helv. Chem. Acta, 56, 1476 (1973)]. When it is desired to use a resin containing a benzhydrylamine moiety as the resin binding site an amino protected amino acid is coupled to the resin through an amide linkage between its α-carboxylic acid and the amino moiety of the resin. This coupling is effected using standard coupling procedures as described below. Many resin-bound amino acids are available commercially.

The α-amino protecting group employed with each amino acid introduced into the polypeptide sequence may be any such protecting group known in the art. Among the classes of amino protecting groups contemplated are: (1) acyl type protecting groups such as formyl, trifluoroacetyl, phthalyl, p-toluenesulfonyl (tosyl), benzenesulfonyl, nitrophenylsulfenyl, tritylsulfenyl, o-nitrophenoxyacetyl, and α-chlorobutyryl; (2) aromatic urethane type protecting groups such as benzyloxycarbonyl and substituted benzyloxycarbonyls such as p-chlorobenzyloxycarbonyl, p-methoxybenzyloxycarbonyl, p-nitrobenzyloxycarbonyl, p-bromobenzyloxycarbonyl, 1-(p-biphenylyl)-1-methylethoxycarbonyl, α-, α-dimethyl-3,5-dimethoxybenzyloxycarbonyl, and benzhydryloxycarbonyl; (3) aliphatic urethane protecting groups such as tert-butyloxycarbonyl (Boc), diisopropylmethoxycarbonyl, isopropyloxycarbonyl, ethoxycarbonyl, and allyloxycarbonyl; (4) cycloalkyl urethane type protecting groups such as cyclopentyloxycarbonyl, adamantyloxycarbonyl, and cyclohexyloxycarbonyl; (5) thio urethane type protecting groups such as phenylthiocarbonyl; (6) alkyl type protecting groups such as triphenylmethyl (trityl) and benzyl (Bzl); (7) trialkylsilane protecting groups such as trimethylsilane. The preferred α-amino protecting group is tert-butyloxycarbonyl (Boc). The use of Boc as an α-amino protecting group for amino acids is described by Bodansky et al. in "The Practice of Peptide Synthesis", Springer-Verlag, Berlin (1984), p. 20.

Following the coupling of the amino protected amino acid to the resin support, the α-amino protecting group is removed using any suitable procedure such as by using trifluoroacetic acid, trifluoroacetic acid in dichloromethane, or HCl in dioxane. The deprotection is carried out at a temperature of between 0°C and room temperature. Other standard cleaving reagents may be used for removal of specific amino protecting groups under conditions well known and appreciated in the art.

After removal and neutralization of the α-amino protecting group the next desired amino-protected amino acid is coupled through a peptide linkage. This deprotection, neutralization and coupling procedure is repeated until a polypeptide of the desired sequence is obtained. Alternatively, multiple amino acid groups may be coupled by the solution method prior to coupling with the resin supported amino acid sequence.

The selection and use of an appropriate coupling reagent is within the skill of the ordinary practitioner in the art. Particularly suitable coupling reagents where the amino acid to be added is Gln, Asn, or Arg are N,N-dicyclohexylcarbodiimide and 1-hydroxybenzotriazole. The use of these reagents prevents nitrile and lactam formation. Other coupling agents are (1) carbodiimides (e.g., N,N-dicyclohexylcarbodiimide and N-ethyl-N'-(γ-dimethylaminopropylcarbodiimide); (3) ketenimines; (4) isoxazolium salts (e.g., N-ethyl-5-phenylisoxazolium-3-sulfonate); (5) monocyclic nitrogen containing heterocyclic amides of aromatic character containing one through four nitrogens in the ring such as imidazolides, pyrazolides, and 1,2,4-triazolides (specific heterocyclic amides that are useful include N,N-carbonyldiimidazole and N,N-carbonyl-di-1,2,4-triazole); (6) alkoxylated acetylene (e.g., ethoxyacetylene); (7) reagents which form a mixed anhydride with the carboxyl moiety of the amino acid (e.g., ethylchloroformate and isobutylchloroformate) or the the symmetrical anhydride of the amino acid to be coupled (e.g., Boc-Ala-o-Ala-Boc); (8) nitrogen containing heterocyclic compounds having a hydroxy group on one ring nitrogen (e.g., N-hydroxyphthalimide, N-hydroxysuccinimide, and 1-hydroxybenzotriazole). Other activating reagents and their use in peptide coupling are described by Kapoor [J. Pharm. Sci., 59, 1-27 (1970)]. The generally preferred coupling method for the amino acids used in the present invention is the use of the symmetrical anhydride as the coupling agent.

The preferred coupling method for Gln, Asn and Arg is to react the protected amino acid, or derivatives or isomers thereof, with N,N-dicyclohexylcarbodiimide and 1-hydroxybenzotriazole (1:1) in N,N-dimethylformamide (DMF) in the presence of the resin or resin-bound amino acid or peptide. The preferred coupling method for other amino acids involves reacting the protected amino acid, or derivative or isomer thereof, with N,N-dicyclohexylcarbodiimide in dichloromethane to form the symmetrical anhydride. The symmetrical anhydride is then introduced into the solid phase reactor containing the resin or resin-bound amino acid or peptide, and the coupling is carried out in a medium of (DMF), or dichloromethane, or DMF: dichloromethane (1:1). A medium of DMF is preferred. The success of the coupling reaction at each stage of the synthesis is monitored by a ninhydrin test as described by Kaiser et al. [Analyt. Biochem. 34, 595 (1970)]. In cases where incomplete coupling occurs, the coupling procedure is repeated. If the coupling is still incomplete, the deprotected amine is capped with a suitable capping reagent to prevent its continued synthesis. Suitable capping reagents and the use thereof are well known and appreciated in the art. Examples of suitable capping reagents are acetic anhydride and acetylimidazole as described by Stewart et al. ["Solid Phase Peptide Synthesis", 2nd Ed., Pierce Chemical Co., Rockford, Ill. (1984), Chapter 2, p. 73].

After the desired amino acid sequence has been obtained, the peptide is cleaved from the resin. This can be effected by procedures which are well known and appreciated in the art, such as by hydrolysis of the ester or amide linkage to the resin. It is preferred to cleave the peptide from the benzhydrylamine resin with a solution of dimethyl sulfide, p-cresol, thiocresol, or anisole in anhydrous hydrogen fluoride. The cleavage reaction is preferably carried out at temperatures between about 0°C and about room temperature, and is allowed to continue preferably from between about 5 minutes to about 5 hours.

As is known in the art of solid phase peptide synthesis, many of the amino acids bear side chain functionalities requiring protection during the preparation of the peptide. The selection and use of an appropriate protecting group for these side chain functionalities is within the ability of those skilled in the art and will depend upon the amino acid to be protected and the presence of other protected amino acid residues in the peptide. The selection of such a side chain protecting group is critical in that it must not be removed during the deprotection and coupling steps of the synthesis. For example, when Boc is used as the α-amino protecting group, the following side chain protecting groups are suitable: p-toluenesulfonyl (tosyl) moieties can be used to protect the amino side chains of amino acids such as Lys and Arg; p-methylbenzyl, acetamidomethyl, benzyl (Bz1), or t-butylsulfonyl moieties can be used to protect the sulfide containing side chains of amino acids such as cysteine, homocysteine, penicillamine and the like or derivatives thereof; benzyl (Bz1) or cyclohexyl ester moieties can be used to protect carboxylic acid side chains of amino acids such as Asp, Glu; a benzyl (Bz1) ether can be used to protect the hydroxy containing side chains of amino acids such as Ser and Thr; and a 2-bromocarbobenzoxy (2Br-Z) moiety can be used to protect the hydroxy containing side chains of amino acids such as Tyr. These side chain protecting groups are added and removed according to standard practices and procedures well known in the art. It is preferred to deprotect these side chain protecting groups with a solution of anisole in anhydrous hydrogen fluoride (1:10). Typically, deprotection of side chain protecting groups is performed after the peptide chain synthesis is complete but these groups can alternatively be removed at any other appropriate time. It is preferred to deprotect these side chains at the same time as the peptide is cleaved from the resin.

The compounds are then isolated and purified by standard techniques. The desired amino acids, derivatives and isomers thereof can be obtained commercially or can be synthesized according to standard practices and procedures well known in the art.

The following specific examples are given to illustrate the preparation of this invention although the scope of compounds is meant to be limiting to the scope of compounds embraced by formula I.

### EXAMPLE 1

### L-Phenylalaninamide, N-[(phenylmethoxy)carbonyl]-L-valyl-N-methoxy-N-methyl

To a suspension of L-phenylalanine, N-[N-[(phenylmethoxy) carbonyl]-L-valyl] (2.5 g, 6.25 mmol) in methylene chloride (25 ml), N-methylmorpholine (1.5 ml) was added. The solution was cooled to -15°C, followed by the addition of isobutylchloroformate (0.8 ml). The solution was stirred for 20 min and N'O-dimethylhydroxylamine HCl (1.0 g) was added. The solution was stirred at -15°C for 1 h, allowed to warm to room temperature and stirred for an additional 3 h. The reaction mixture was poured into dil. NaHCO₃ and extracted with ethyl acetate (3 x 75 ml). The combined extracts were dried over Na₂SO₄, the solvent was removed *in vacuo* and the crude product was loaded onto a silica gel column for purification. The expected product was eluted with 75% EtOAc/hexane to yield 1.8 g.

### EXAMPLE 2

### L-N-(Phenylmethoxy)carbonyl-phenylalaninamide-N'-methoxy-N'-methyl

To a solution of L-N-(phenylmethoxy)carbonyl-phenylalanine (25 g, 0.084 mol) in methylene chloride (300 ml), N-methylmorpholine (18.4 ml, 0.167 mol) was added. The mixture was cooled to -15°C and isobutylchloroformate (10.8 ml, 83.6 mmol) was added. The mixture was stirred at -15°C for 15 min followed by the addition of N,O-dimethylhydroxylamine HCl (8.5 g). The mixture was stirred at -15°C for 1 h, allowed to warm to room temperature and stirred for 3 h. The reaction mixture was poured into H₂O (300 ml) and the aqueous phase was extracted with methylene chloride (2 x 150 ml). The combined organic extracts were dried over Na₂SO₄, the volume was reduced to 100 ml and filtered through silica gel (2 inch). The silica gel was washed with methylene chloride (200 ml) and the solvent was removed from the combined filtrates to yield 26.14 g of the expected product.

### EXAMPLE 3

### L-Phenylalaninal, N[(phenylmethoxy)carbonyl]-L-valyl

A solution of L-phenylalaninamide, N[(phenyl-methoxy) carbonyl]-L-valyl-N'-methoxy-N'-methyl (3 g, 6.8 mol) in tetrahydrofuran (50 ml) was cooled to 0°C and LAH (250 mg) was added. The mixture was stirred at 0°C for 30 min and quenched by the addition of 10% potassium hydrogen sulfate. The mixture was poured into H₂O (400 ml) and the aqueous phase was extracted with ethyl acetate (3 x 150 ml). The combined organic extracts were dried over MgSO₄ and the solvent was removed *in* *vacuo*. The crude product was loaded onto silica gel for purification and the product was eluted with 55% EtOAc/hexane to yield 1.6 g of the expected compound.

### EXAMPLE 4

### 2-[L-N-(Phenylmethoxycarbonyl)amino)phenylalaninyl]-1,3-dithiane

To a solution of 1,3-dithiane (6.0 g, 0.05 mol) in tetrahydrofuran (150 ml) at -30°C, n-butyllithium (27.5 ml of 2.0 M n-butyllithium in pentane, 0.055 mol) is added. The mixture is stirred for 2 h and L-N-[(phenylmethoxy)carbonyl]-N'-methoxy-N'-methylphenylalaninamide (3.42 g, 10.0 mmol) is dissolved in tetrahydrofuran (15 ml) and added. The reaction mixture is stirred at 0°C for 24 h, poured into H₂O and extracted with diethyl ether. The combined organic extracts are washed with H₂O, saturated NaCl and dried over Na₂SO₄. The solvent is removed *in vacuo* and the crude product is purified by flash chromatography on silica gel.

### EXAMPLE 5

### 3-[((Phenylmethoxy)carbonyl)amino]-4-phenyl-2-oxobutyraldehyde

To a solution of 2-[L-N-(phenylmethoxycarbonyl)amino) phenylalaninyl]-1,3-dithiane (387 mg, 1.0 mmol) in a mixture of acetonitrile/H₂O (9:1) (10 ml), bis(trifluoroacetoxy)iodobenzene (644 mg, 1.5 mmol) is added. The reaction mixture is stirred at room temperature until completion as determined by thin layer chromatography, poured into saturated aqueous sodium bicarbonate and extracted with diethyl ether. The combined organic extracts are dried over Na₂SO₄, the solvent is removed *in vacuo* and the crude product is purified by flash chromatography on silica gel.

The foregoing describes in detail the generic and specific aspects of the scope of the invention as well as the manner of making and using the invention. In addition thereto, although such procedures are known in the art, references setting forth state of the art procedures by which the compounds may be evaluated for their biochemical effects is also included herein.

For example, human elastase is assayed *in vitro* using chromophoric peptides, succinylalanylalanylalanyl-p-nitro-anilide, methoxysuccinylalanylalanylprolylvalyl-p-nitroanilide, and others, all of which are available commercially. The assay buffer, pH 8.0, and assay techniques are similar to those described by Lottenberg et al. The enzyme is purified from human sputum, although recently it has become commercially available. Kinetic characterization of immediate inhibitors is by means of the Dixon plot, whereas the characterization of slow- and/or tight-binding inhibitors used data analysis techniques reviewed by Williams and Morrison.

Similarly, the other proteases are assayed and effects of inhibitors are assessed *in vitro* by similar spectroscopic techniques: cathepsin G; thrombin; chymotrypsin; trypsin; plasmin; Cl-esterase; urokinase; plasminogen activator; acrosin; β-lactamase; cathepsin B; pepsin; cathepsin D and leucine aminopeptidase. Pseudomonas elastase is measured in a coupled assay procedure using a human elastase substrate and microsomal aminopeptidase.

Radiometric assays of angiotensin I-converting enzyme and enkephalinase and their inhibitors are based on the procedure of Ryan and use tritiated substrate purchased from Ventrex Laboratories, Inc. Radioimmunoassay is used for studies with renin. C3-convertase is measured as described by Tack et al.

By following the technique referred to above, as well as by utilization of other known techniques, as well as by comparison with compounds known to be useful for treatment of the above-mentioned disease states, it is believed that adequate material is available to enable one of ordinary skill in the art to practice the invention. Of course, in the end-use application of the compounds of this invention, the compounds are preferably formulated into suitable pharmaceutical preparations such as tablets, capsules or elixers, for oral administration or in sterile solutions or suspensions for parenteral administration. The compounds of this invention can be administered to patients (animals and human) in need of such treatment in a dosage range of 0.01-10 mg per kg of body weight per day. As stated above, the dose will vary depending on severity of disease, weight of patient and other factors which a person skilled in the art will recognize.

Typically the compounds described above are formulated into pharmaceutical compositions as discussed below.

About 10 to 500 mg of a compound or mixture of compounds of Formula I or a physiologically acceptable salt is compounded with a physiologically acceptable vehicle, carrier, excipient, binder, perservative, stabilizer, flavor, etc., in a unit dosage form as called for by accepted pharmaceutical practice. The amount of active substance in these compositions or preparations is such that a suitable dosage in the range indicated is obtained.

Illustrative of the adjuvants which may be incorporated in tablets, capsules and the like are the following: a binder such as gum tragacanth, acacia, corn starch or gelatin; an excipient such as microcrystalline cellulose; a disintegrating agent such as corn starch, pregelatinized starch, alginic acid and the like; a lubricant such as magnesium stearate; a sweetening agent such as sucrose, lactose or saccharin; a flavoring agent such as peppermint, oil of wintergreen or cherry. When the dosage unit form is a capsule, it may contain in addition to materials of the above type, a liquid carrier such as fatty oil. Various other materials may be present as coatings or to otherwise modify the physical form of the dosage unit. For instance, tablets may be coated with shellac, sugar or both. A syrup or elixir may contain the active compound, sucrose as a sweetening agent, methyl and propyl parabens as preservatives, a dye and a flavoring such as cherry or orange flavor.

Sterile compositions for injection can be formulated according to conventional pharmaceutical practice by dissolving or suspending the active substance in a vehicle such as water for injection, a naturally occurring vegetable oil like sesame oil, coconut oil, peanut oil, cottonseed oil, etc. or a synthetic fatty vehicle like ethyl oleate or the like. Buffers, preservatives, antioxidants and the like can be incorporated as required.

While the invention has been described in connection with specific embodiments thereof, it will be understood that it is capable of further modifications and this application is intended to cover any variations, uses or adaptations of the invention following, in general, the principles of the invention and including such departures from the present disclosure as come within known or customary practice within the art to which the invention pertains and as may be applied to the essential features hereinbefore set forth, and as follows in the scope of the appended claims.

## Claims

1. Use of compounds of formula (Iu):
R₁NHCHR₂C(O)H (Iu)
in which
R₁ is a Group K protecting group, P₂P₃ or P₂P₃P_{g} ;
P_{g} being a Group K protecting group ;
P₂ is an α-amino acid of Groups E and F or Lys ;
P₃ is deleted or is an α-amino acid of Groups B, E or F,
R₂ is H, the residue of an α-amino acid of Groups E, F and J or -A-Si-R₇R₈R₉, C₁₋₇ alkyl, benzyl, phenethyl or naphthyl ;
The said groups B, E, F, J, K being as follows:
B: Glu, Asp ;
E: Ala, β-Ala, Leu, Ile, Val, n-Val, β-Val, Met,
n-Leu and their N-methyl derivatives ;
F: Phe, Tyr, O-Methyl Tyrosine, (3-pyrazolyl)Ala, (4-pyrimidinyl)Ala, Trp, Nal(1), and their N-methyl derivatives ;
J: 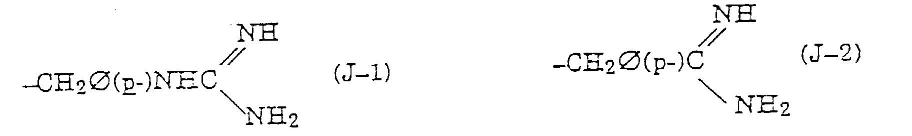 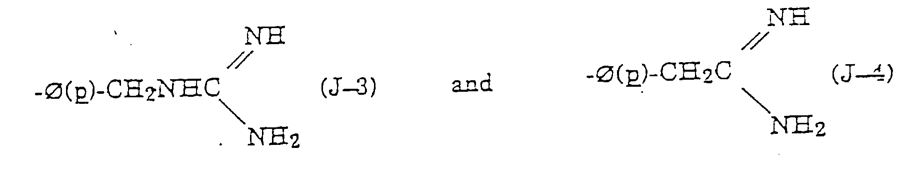 with Ø representing phenyl ;
K: Acetyl (Ac), Succinyl (Suc), Methoxysuccinyl (H₃COSuc), Benzoyl (Bz), t-Butyloxycarbonyl (Boc), Carbobenzoxy (CBZ), Tosyl (Ts), Dansyl (DNS), Iso-valeryl (Iva), Methoxysuccinyl (MeOSuc), 1-Adamantanesulphonyl (AdSO₂), 1-Adamantaneacetyl (AdAc), 2-Carboxybenzoyl (2-CBZ), Phenylacetyl, t-Butylacetyl (Tba), bis [(1-naphthyl)methyl]acetyl (BNMA),
A is a C₁₋₆ alkylene, each of R₇, R₈ and R₉ are C₁₋₁₀ alkyl, benzyl or phenethyl,
for the preparation of medicaments useful as inhibitors of calpain.

2. Use of compounds of formula (Iu) :
R₁NHCHR₂C(O)H (Iu)
in which
R₁ is a Group K protecting group, P₂P₃ or P₂P₃P_{g} ;
P_{g} being a Group K protecting group ;
P₂ is an α-amino acid of Groups E and F or Lys;
P₃ is deleted or is an α-amino acid of Groups B, E or F,
R₂ is H, the residue of an α-amino acid of Groups E, F and J or -A-Si-R₇R₈R₉, C₁₋₇ alkyl, benzyl, phenethyl or naphthyl ;
The said groups B, E, F, J, K being as follows:
B: Glu, Asp ;
E: Ala, β-Ala, Leu, Ile, Val, n-Val, β-Val, Met, n-Leu and their N-methyl derivatives ;
F: Phe, Tyr, O-Methyl Tyrosine, (3-pyrazolyl)Ala, (4-pyrimidinyl)Ala, Trp, Nal(1), and their N-methyl derivatives ;
J: 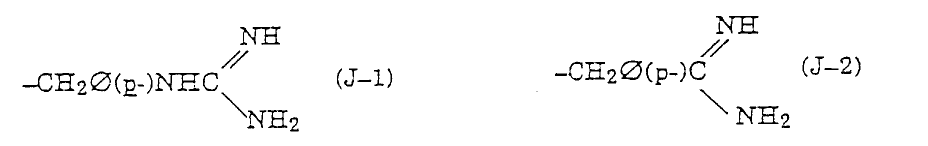 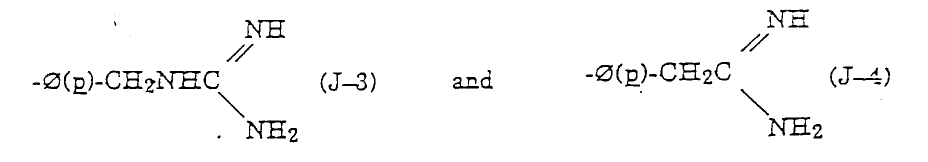 with Ø representing phenyl ;
K: Acetyl (Ac), Succinyl (Suc), Methoxysuccinyl (H₃COSuc), Benzoy (Bz), t-Butyloxycarbonyl (Boc), Carbobenzoxy (CBZ), Tosyl (Ts), Dansyl (DNS), Iso-valeryl (Iva), Methoxysuccinyl (MeOSuc), 1-Adamantanesulphonyl (AdSO₂), 1-Adamantaneacetyl (AdAc), 2-Carboxybenzoyl (2-CBZ), Phenylacetyl, t-Butylacetyl (Tba), bis [(1naphthyl)methyl]acetyl (BNMA),
A is a C₁₋₆ alkylene, each of R₇, R₈ and R₉ are C₁₋₁₀ alkyl, benzyl or phenethyl,
for the preparation of medicaments useful for inhibiting calpain-induced and/or cathepsin B-induced breakdown of cytoskeleton.

3. Use of compounds of formula (Iu) :
R₁NHCHR₂C(O)H (Iu)
in which
R₁ is a Group K protecting group, P₂P₃ or P₂P₃P_{g} ;
P_{g} being a Group K protecting group ;
P₂ is an α-amino acid of Groups E and F or Lys ;
P₃ is deleted or is an α-amino acid of Groups B, E or F,
R₂ is H, the residue of an α-amino acid of Groups E, F and J or -A-Si-R₇R₈R₉, C₁₋₇ alkyl, benzyl, phenethyl or naphthyl ;
The said groups B, E, F, J, K being as follows:
B: Glu, Asp ;
E: Ala, β-Ala, Leu, Ile, Val, n-Val, β-Val, Met,
n-Leu and their N-methyl derivatives ;
F: Phe, Tyr, O-Methyl Tyrosine, (3-pyrazolyl)Ala, (4-pyrimidinyl)Ala, Trp, Nal(1), and their N-methyl derivatives ;
J: 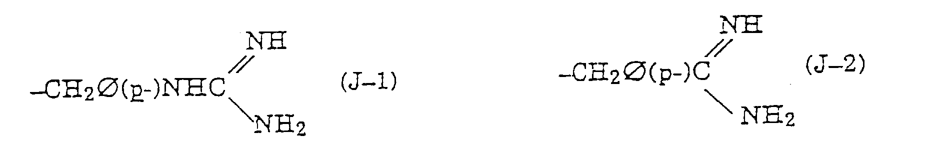 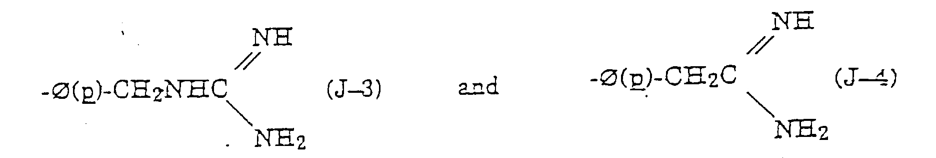 with Ø representing phenyl ;
K: Acetyl (Ac), Succinyl (Suc), Methoxysuccinyl (H₃COSuc), Benzoyl (Bz), t-Butyloxycarbonyl (Boc), Carbobenzoxy (CBZ), Tosyl (Ts), Dansyl (DNS), Iso-valeryl (Iva), Methoxysuccinyl (MeOSuc), 1-Adamantanesulphonyl (AdSO₂), 1-Adamantaneacetyl (AdAc), 2-Carboxybenzoyl (2-CBZ), Phenylacetyl, t-Butylacetyl (Tba), bis [(1-naphthyl)methyl]acetyl (BNMA),
A is a C₁₋₆ alkylene, each of R₇, R₈ and R₉ are C₁₋₁₀ alkyl, benzyl or phenethyl,
for the preparation of medicaments useful for inhibiting calpain-induced and/or cathepsin B-induced red blood cell lysis.

4. Use of compounds of formula (Iu) :
R₁NHCHR₂C(O)H (Iu)
in which
R₁ is a Group K protecting group, P₂P₃ or P₂P₃P_{g} ;
P_{g} being a Group K protecting group ;
P₂ is an α-amino acid of Groups E and F or Lys;
P₃ is deleted or is an α-amino acid of Groups B, E or F,
R₂ is H, the residue of an α-amino acid of Groups E, F and J or -A-Si-R₇R₈R₉, C₁₋₇ alkyl, benzyl, phenethyl or naphthyl ;
The said groups B, E, F, J, K being as follows:
B: Glu, Asp;
E: Ala, β-Ala, Leu, Ile, Val, n-Val, β-Val, Met, n-Leu and their N-methyl derivatives ;
F: Phe, Tyr, O-Methyl Tyrosine, (3-pyrazolyl)Ala, (4-pyrimidinyl)Ala, Trp, Nal(1), and their N-methyl derivatives ;
J: 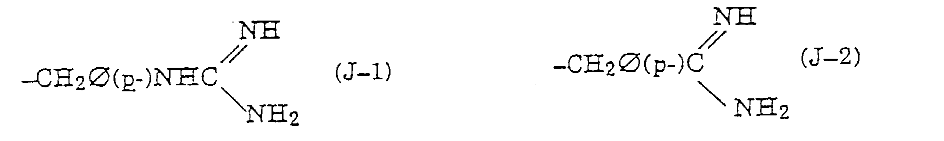 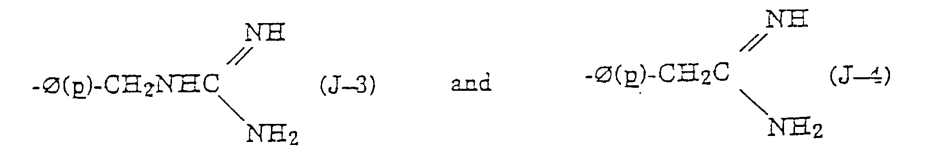 with Ø representing phenyl ;
K: Acetyl (Ac), Succinyl (Suc), Methoxysuccinyl (H₃COSuc), Benzoyl (Bz), t-Butyloxycarbonyl (Boc), Carbobenzoxy (CBZ), Tosyl (Ts), Dansyl (DNS), Iso-valeryl (Iva), Methoxysuccinyl (MeOSuc), 1-Adamantanesulphonyl (AdSO₂), 1-Adamantaneacetyl (AdAc), 2-Carboxybenzoyl (2-CBZ), Phenylacetyl, t-Butylacetyl (Tba), bis [(1-naphthyl)methyl]acetyl (BNMA),
A is a C₁₋₆ alkylene, each of R₇, R₈ and R₉ are C₁₋₁₀ alkyl, benzyl or phenethyl,
for the preparation of medicaments useful for inhibiting calpain-induced and/or cathepsin B-induced intracellular proteolysis.

5. Use according to claim 4, for the preparation of medicaments useful for the treatment of strokes.

6. Use of the compounds of formula Iu according to any one of claims 1 to 5, in which R₁ is a protecting group selected from CBZ, Bz or Ac.

7. Use of the compounds of formula Iu according to any one of claims 1 to 5, in which P₂ is Ala, Phe or Lys.

8. Use of the compounds of formula Iu according to claim 4, in which P₃ is deleted.

9. Use of the compounds of formula Iu according to claim 4, in which R₂ is
cyclohexylmethyl (CHM), naphthyl (NAP),
trimethylsilylmethyl (TMSM),
benzyldimethylsilylmethyl (BDMS-M), Lys or Phe.

10. Use according to any one of claims 1 to 5, in which the compound of formula Iu is CBZ-Val-Phe(H).

## Patentansprüche

1. Verwendung von Verbindungen der Formel (Iu):
R₁NHCHR₂C(O)H (Iu),
wobei
R₁ eine Rest K-Schutzgruppe, P₂P₃ oder P₂P₃P_{g} ist:
wobei P_{g} eine Rest K-Schutzgruppe ist;
P₂ eine α-Aminosäure der Gruppen E und F oder Lys ist,
P₃ deletiert ist oder eine α-Aminosäure der Gruppen B, E oder F ist,
R₂ H, der Rest einer A-Aminosäure der Gruppen E, F und J oder -A-Si-R₇R₈R₉. C₁₋₇-Alkyl, Benzyl, Phenethyl oder Naphthyl ist:
wobei die Reste B, E, F, J, K die nachstehende Bedeutung haben:
B: Glu, Asp;
E: Ala, β-Ala, Leu, Ile, Val, n-Val, β-Val, Met, n-Leu und ihre N-Methyl-Derivate:
F: Phe, Tyr, O-Methyl-Tyrosin, (3-Pyrazolyl)-Ala, (4-Pyrimidynyl)-Ala, Trp, Nal(1) und ihre N-Methyl-Derivate:
J: 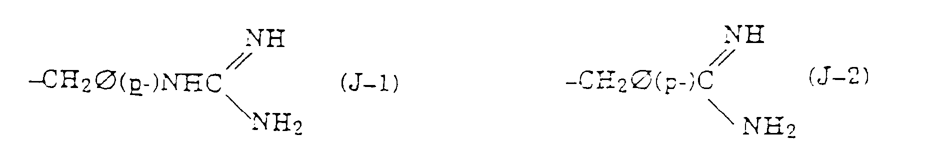 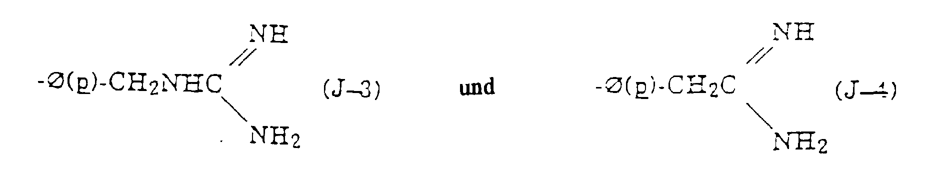 wobei Ø Phenyl darstellt;
K: Acetyl (Ac), Succinyl (Suc), Methoxysuccinyl (H₃COSuc), Benzoyl (Bz), t-Butyloxycarbonyl (Boc), Carbobenzoxy (CBZ), Tosyl (Ts), Dansyl (DNS), Isovaleryl (Iva), Methoxysuccinyl (MeOSuc), 1-Adamantansulfonyl (AdSO₂), 1-Adamantanacetyl (AdAc), 2-Carboxybenzoyl (2-CBZ), Phenylacetyl, t-Butylacetyl (Tba), Bis[(1-naphthyl)methyl]acetyl (BNMA),
A C₁₋₆-Alkylen ist, jeder der Reste R₇, R₈ und R₉ C₁₋₁₀-Akyl Benzyl oder Phenethyl ist, zur Herstellung von Medikamenten, die als Hemmstoffe von Calpain geeignet sind.

2. Verwendung von Verbindungen der Formel (Iu):
R₁NHCHR₂C(O)H (Iu),
wobei
R₁ eine Rest K-Schutzgruppe, P₂P₃ oder P₂P₃P_{g} ist:
wobei P_{g} eine Rest K-Schutzgruppe ist;
P₂ eine α-Aminosäure der Gruppen E und F oder Lys ist,
P₃ deletien ist oder eine α-Aminosäure der Gruppen B, E oder F ist,
R₂ H, der Rest einer α-Aminosäure der Gruppen E, F und J oder -A-Si-R₇R₈R₉, C₁₋₇-Alkyl Benzyl, Phenethyl oder Naphthyl ist:
wobei die Reste B, E, F, J, K die nachstehende Bedeutung haben:
B: Glu, Asp;
E: Ala, β-Ala, Leu, Ile, Val, n-Val, β-Val, Met, n-Leu und ihre N-Methyl-Derivate:
F: Phe, Tyr, O-Methyl-Tyrosin, (3-Pyrazolyl)-Ala, (4-Pyrimidinyl)-Ala, Trp, Nal(1) und ihre N-Methyl-Derivate:
J: 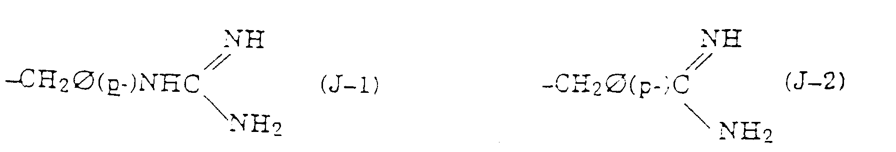 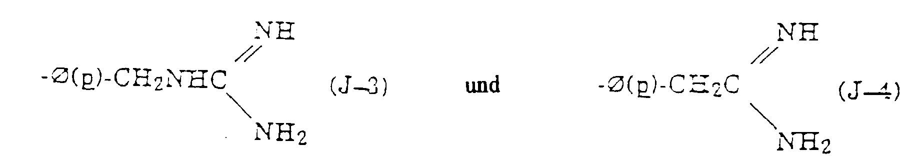 wobei Ø Phenyl darstellt;
K: Acetyl (Ac), Succinyl (Suc), Methoxysuccinyl (H₃COSuc), Benzoyl (Bz), t-Butyloxycarbonyl (Boc), Carbobenzoxy (CBZ), Tosyl (Ts), Dansyl (DNS), Isovaleryl (Iva), Methoxysuccinyl (MeOSuc), 1-Adamantansulfonyl (AdSO₂), 1-Adamantanacetyl (AdAc), 2-Carboxybenzoyl (2-CBZ), Phenylacetyl, t-Butylacetyl (Tba), Bis[(1-naphthyl)methyl]acetyl (BNMA),
A C₁₋₆-Alkylen ist, jeder der Reste R₇, R₈ und R₉ C₁₋₁₀-Alkyl, Benzyl oder Phenethyl ist, zur Herstellung von Medikamenten, die zur Hemmung des Calpain-induzierten und/oder Cathepsin B-induzierten Abbaus von Cytoskeleton geeignet sind.

3. Verwendung von Verbindungen der Formel (Iu):
R₁NHCHR₂C(O)H (Iu)
wobei
R₁ eine Rest K-Schutzgruppe, P₂P₃ oder P₂P₃P_{g} ist;
wobei P_{g} eine Rest K-Schutzgruppe ist;
P₂ eine α-Aminosäure der Gruppen E und F oder Lys ist,
P₃ deletiert ist oder eine α-Aminosäure der Gruppen B, E oder F ist,
R₂ H, der Rest einer α-Aminosäure der Gruppen E, F und J oder -A-Si-R₇R₈R₉, C₁₋₇-Alkyl, Benzyl, Phenethyl oder Naphthyl ist:
wobei die Reste B, E, F, J, K die nachstehende Bedeutung haben:
B: Glu, Asp;
E: Ala, β-Ala, Leu, Ile, Val, n-Val, β-Val, Met, n-Leu und ihre N-Methyl-Derivate:
F: Phe, Tyr, O-Methyl-Tyrosin, (3-Pyrazolyl)-Ala, (4-Pyrimidinyl)-Ala, Trp, Nal(1) und ihre N-Methyl-Derivate;
J: 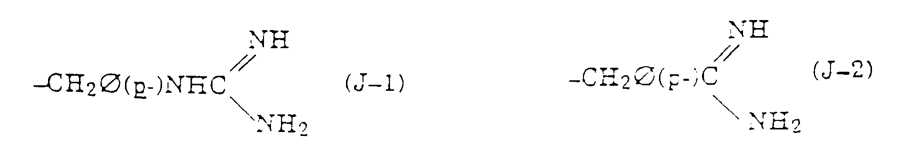 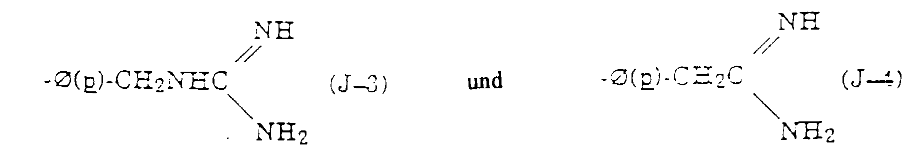 wobei Ø Phenyl darstellt;
K: Acetyl (Ac), Succinyl (Suc), Methoxysuccinyl (H₃COSuc), Benzoyl (Bz), t-Butyloxycarbonyl (Boc), Carbobenzoxy (CBZ), Tosyl (Ts), Dansyl (DNS), Isovaleryl (Iva), Methoxysuccinyl (MeOSuc), 1-Adamantansulfonyl (AdSO₂), 1-Adamantanacetyl (AdAc), 2-Carboxybenzoyl (2-CBZ), Phenylacetyl, t-Butylacetyl (Tba), Bis[(1-naphthyl)methyl]acetyl (BNMA),
A C₁₋₆-Alkylen ist, jeder der Reste R₇, R₈ und R₉ C₁₋₁₀-Alkyl, Benzyl oder Phenethyl ist, zur Herstellung von Medikamenten, die zur Hemmung der Calpain-induzierten und/oder Cathepsin B induzierten Lysis roter Blutzellen geeignet sind.

4. Verwendung von Verbindungen der Formel (Iu):
R₁NHCHR₂C(O)H (Iu),
wobei
R₁ eine Rest K-Schutzgruppe, P₂P₃ oder P₂P₃P_{g} ist;
wobei P_{g} eine Rest K-Schutzgruppe ist;
P₂ eine α-Aminosäure der Gruppen E und F oder Lys ist,
P₃ deletiert ist oder eine α-Aminosäure der Gruppen B, E oder F ist,
R₂ H, der Rest einer α-Aminosäure der Gruppen E, F und J oder -A-Si-R₇R₈R₉, C₁₋₇-Alkyl Benzyl, Phenethyl oder Naphthyl ist;
wobei die Reste B, E, F, J, K die nachstehende Bedeutung haben:
B: Glu, Asp;
E: Ala, β-Ala, Leu, Ile, Val, n-Val, β-Val, Met, n-Leu und ihre N-Methyl-Derivate;
F: Phe, Tyr, O-Methyl-Tyrosin, (3-Pyrazolyl)-Ala, (4-Pyrimidinyl)-Ala, Trp, Nal(1) und ihre N-Methyl-Derivate;
J: 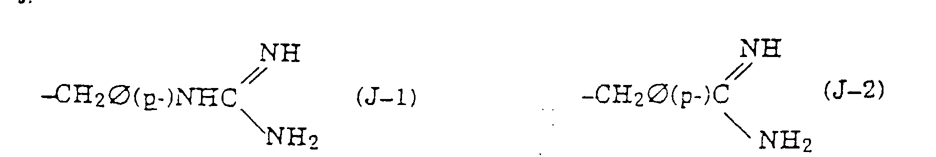 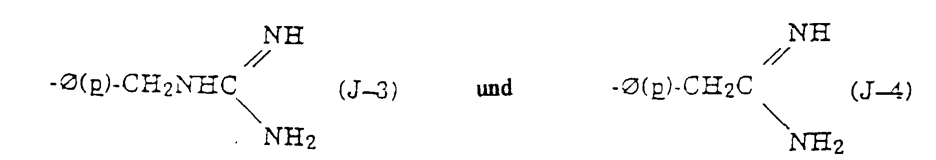 wobei Ø Phenyl darstellt;
K: Acetyl (Ac), Succinyl (Suc), Methoxysuccinyl (H₃COSuc), Benzoyl (Bz), t-Butyloxycarbonyl (Boc), Carbobenzoxy (CBZ), Tosyl (Ts), Dansyl (DNS), Isovaleryl (Iva), Methoxysuccinyl (MeOSuc), 1-Adamantansulfonyl (AdSO₂), 1-Adamantanacetyl (AdAc), 2-Carboxybenzoyl (2-CBZ), Phenylacetyl, t-Butylacetyl (Tba), Bis[(1-naphthyl)methyl]acetyl (BNMA),
A C₁₋₆-Alkylen ist, jeder der Reste R₇, R₈ und R₉ C₁₋₁₀-Alkyl, Benzyl oder Phenethyl ist, zur Herstellung von Medikamenten, die zur Hemmung der Calpain-induzierten und/oder Cathepsin β-induzierten intrazellulären Proteolyse geeignet sind.

5. Verwendung nach Anspruch 4 für die Herstellung von Medikamenten, die zur Behandlung von Schlaganfällen geeignet sind.

6. Verwendung der Verbindungen der Formel Iu nach einem der Ansprüche 1 bis 5, wobei R₁ eine Schutzgruppe ist, ausgewählt aus CBZ, Bz oder Ac.

7. Verwendung der Verbindungen der Formel Iu nach einem der Ansprüche 1 bis 5, wobei P₂ Ala, Phe oder Lys ist.

8. Verwendung der Verbindungen der Formel Iu nach Anspruch 4, wobei P₃ deletiert ist.

9. Verwendung der Verbindungen der Formel Iu nach Anspruch 4, wobei R₂ Cyclohexylmethyl (CHM), Naphthyl (NAP), Trimethylsilylmethyl (TMSM), Benzyldimethylsilylmethyl (BDMS-M), Lys oder Phe ist.

10. Verwendung nach einem der Ansprüche 1 bis 5, wobei die Verbindung der Formel Iu CBZ-Val-Phe(H) ist.

## Revendications

1. Utilisation de composés de formule (Iu) :
R₁NHCHR₂C(O)H (Iu)
dans laquelle
R₁ est un groupe protecteur du groupe K, P₂P₃ ou P₂P₃P_{g} ;
P_{g} étant un groupe protecteur du groupe K ;
P₂ est un α-aminoacide des groupes E et F ou Lys ;
P₃ est supprimé ou est un α-aminoacide des groupes B, E ou F,
R₂ est H, le résidu d'un α-aminoacide des groupes E, F et J ou -A-Si-R₇R₈R₉, alkyle en C₁-C₇, benzyle, phénéthyle ou naphtyle ;
lesdits groupes B, E, F, J, K étant les suivants :
B: Glu, Asp ;
E: Ala, β-Ala, Leu, Ile, Val, n-Val, β-Val, Met,
n-Leu et leurs dérivés N-méthylés ;
F: Phe, Tyr, O-méthyltyrosine, (3-pyrazolyl)Ala, (4-pyrimidinyl)Ala, Trp, Nal(1), et leurs dérivés N-méthylés ;
J : 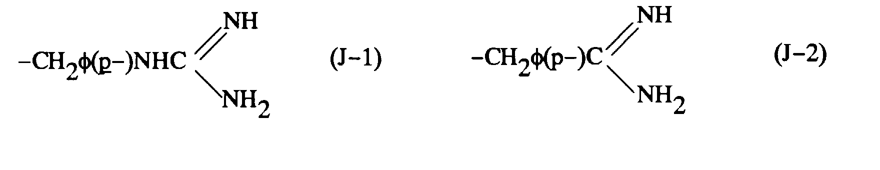 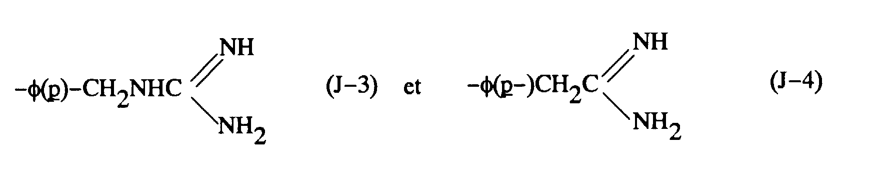 φ représentant phényle ;
K: acétyle (Ac), succinyle (Suc), méthoxysuccinyle (H₃COSuc), benzoyle (Bz), t-butyloxycarbonyle (Boc), carbobenzoxy (CBZ), tosyle (Ts), dansyle (DNS), isovaléryle (Iva), méthoxysuccinyle (MeOSuc), 1-adamantanesulfonyle (AdSO₂), 1-adamantaneacétyle (AdAc), 2-carboxybenzoyle (2-CBZ), phénylacétyle, t-butylacétyle (Tba), bis [(1-naphtyl)méthyl]acétyle (BNMA),
A est un alkylène en C₁-C₆, R₇, R₈ et R₉ sont chacun alkyle en C₁-C₁₀, benzyle ou phénéthyle,
pour la préparation de médicaments utiles comme inhibiteurs de la calpaine.

2. Utilisation de composés de formule (Iu) :
R₁NHCHR₂C(O)H (Iu)
dans laquelle
R₁ est un groupe protecteur du groupe K, P₂P₃ ou P₂P₃P_{g} ;
P_{g} étant un groupe protecteur du groupe K ;
P₂ est un α-aminoacide des groupes E et F ou Lys ;
P₃ est supprimé ou est un α-aminoacide des groupes B, E ou F;
R₂ est H, le résidu d'un α-aminoacide des groupes E, F et J ou -A-Si-R₇R₈R₉, alkyle en C₁-C₇, benzyle, phénéthyle ou naphtyle ;
lesdits groupes B, E, F, J, K étant les suivants :
B: Glu, Asp;
E: Ala, β-Ala, Leu, Ile, Val, n-Val, β-Val, Met, n-Leu et leurs dérivés N-méthylés;
F: Phe, Tyr, O-méthyltyrosine, (3-pyrazolyl)Ala, (4-pyrimidinyl)Ala, Trp, Nal(1), et leurs dérivés N-méthylés ;
J: 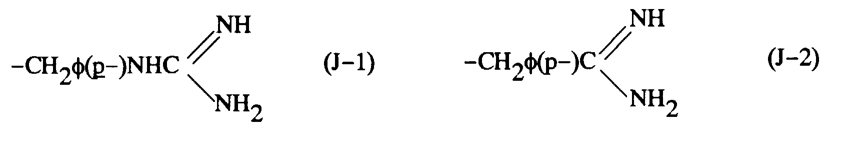 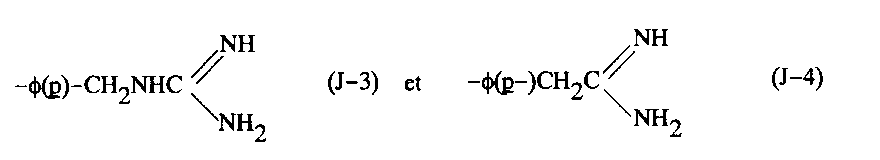 φ représentant phényle ;
K: acétyle (Ac), succinyle (Suc), méthoxysuccinyle (H₃COSuc), benzoyle (Bz), t-butyloxycarbonyle (Boc), carbobenzoxy (CBZ), tosyle (Ts), dansyle (DNS), isovaléryle (Iva), méthoxysuccinyle (MeOSuc), 1-adamantanesulfonyle (AdSO₂), 1-adamantaneacétyle (AdAc), 2-carboxybenzoyle (2-CBZ), phénylacétyle, t-butylacétyle (Tba), bis [(1-naphtyl)méthyl]acétyle (BNMA), A est un alkylène en C₁-C₆, R₇, R₈ et R₉ sont chacun alkyle en C₁-C₁₀, benzyle ou phénéthyle,
pour la préparation de médicaments utiles pour inhiber une rupture du cytosquelette induite par la calpaïne et/ou induite par la cathepsine B.

3. Utilisation de composés de formule (Iu) :
R₁NHCHR₂C(O)H (Iu)
dans laquelle
R₁ est un groupe protecteur du groupe K, P₂P₃ ou P₂P₃P_{g} ;
P_{g} étant un groupe protecteur du groupe K ;
P₂ est un α-aminoacide des groupes E et F ou Lys ;
P₃ est supprimé ou est un α-aminoacide des groupes B, E ou F,
R₂ est H, le résidu d'un α-aminoacide des groupes E, F et J ou -A-Si-R₇R₈R₉, alkyle en C₁-C₇, benzyle, phénéthyle ou naphtyle ;
lesdits groupes B, E, F, J, K étant les suivants :
B: Glu, Asp;
E: Ala, β-Ala, Leu, Ile, Val, n-Val, β-Val, Met, n-Leu et leurs dérivés N-méthylés ;
F: Phe, Tyr, O-méthyltyrosine, (3-pyrazolyl)Ala, (4-pyrimidinyl)Ala, Trp, Nal(1), et leurs dérivés N-méthylés ;
J : 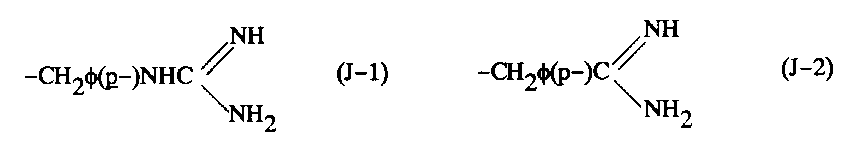 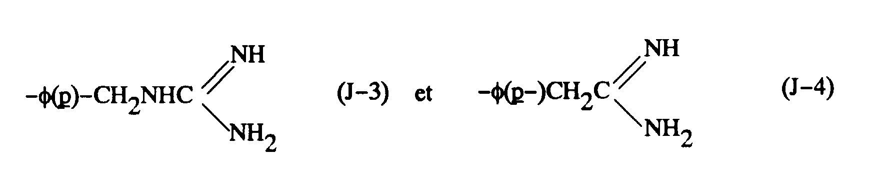 φ représentant phényle ;
K: acétyle (Ac), succinyle (Suc), méthoxysuccinyle (H₃COSuc), benzoyle (Bz), t-butyloxycarbonyle (Boc), carbobenzoxy (CBZ), tosyle (Ts), dansyle (DNS), isovaléryle (Iva), méthoxysuccinyle (MeOSuc), 1-adamantanesulfonyle (AdSO₂), 1-adamantaneacétyle (AdAc), 2-carboxybenzoyle (2-CBZ), phénylacétyle, t-butylacétyle (Tba), bis [(1-naphtyl)méthyl]acétyle (BNMA), A est un alkylène en C₁-C₆, R₇, R₈ et R₉ sont chacun alkyle en C₁-C₁₀, benzyle ou phénéthyle,
pour la préparation de médicaments utiles pour inhiber la lyse des globules rouges induite par la calpaïne et/ou induite par la cathepsine B.

4. Utilisation de composés de formule (Iu) :
R₁NHCHR₂C(O)H (Iu)
dans laquelle
R₁ est un groupe protecteur du groupe K, P₂P₃ ou P₂P₃P_{g} ;
P_{g} étant un groupe protecteur du groupe K ;
P₂ est un α-aminoacide des groupes E et F ou Lys ;
P₃ est supprimé ou est un α-aminoacide des groupes B, E ou F,
R₂ est H, le résidu d'un α-aminoacide des groupes E, F et J ou -A-Si-R₇R₈R₉, alkyle en C₁-C₇, benzyle, phénéthyle ou naphtyle ;
lesdits groupes B, E, F, J, K étant les suivants :
B: Glu, Asp ;
E: Ala, β-Ala, Leu, Ile, Val, n-Val, β-Val, Met,
n-Leu et leurs dérivés N-méthylés ;
F: Phe, Tyr, O-méthyltyrosine, (3-pyrazolyl)Ala, (4-pyrimidinyl)Ala, Trp, Nal(1), et leurs dérivés N-méthylés ;
J : 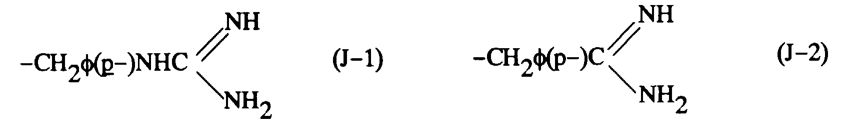 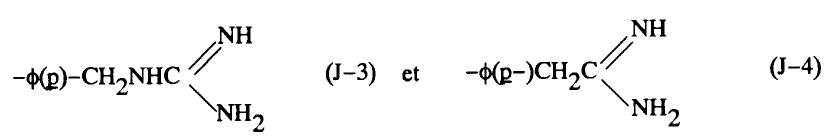 φ représentant phényle ;
K: acétyle (Ac), succinyle (Suc), méthoxysuccinyle (H₃COSuc), benzoyle (Bz), t-butyloxycarbonyle (Boc), carbobenzoxy (CBZ), tosyle (Ts), dansyle (DNS), isovaléryle (Iva), méthoxysuccinyle (MeOSuc), 1-adamantanesulfonyle (AdSO₂), 1-adamantaneacétyle (AdAc), 2-carboxybenzoyle (2-CBZ), phénylacétyle, t-butylacétyle (Tba), bis [(1-naphtyl)méthyl]acétyle (BNMA), A est un alkylène en C₁-C₆, R₇, R₈ et R₉ sont chacun alkyle en C₁-C₁₀, benzyle ou phénéthyle,
pour la préparation de médicaments utiles pour inhiber la protéolyse intracellulaire induite par la calpaïne et/ou induite par la capthesine B.

5. Utilisation selon la revendication 4 pour la préparation de médicaments utiles pour le traitement des ictus.

6. Utilisation des composés de formule (Iu) selon l'une quelconque des revendications 1 à 5, dans lesquels R₁ est un groupe protecteur choisi parmi CBZ, Bz ou Ac.

7. Utilisation des composés de formule lu selon l'une quelconque des revendications 1 à 5, dans lesquels P₂ est Ala, Phe ou Lys.

8. Utilisation des composés de formule Iu selon la revendication 4, dans lesquels P₃ est supprimé.

9. Utilisation des composés de formule Iu selon la revendication 4, dans lesquels R₂ est
cyclohexylméthyle (CHM), naphtyle (NAP),
triméthylsilylméthyle (TMSM),
benzyldiméthylsilylméthyle (BDMS-M), Lys ou Phe.

10. Utilisation selon l'une quelconque des revendications 1 à 5 dans laquelle le composé de formule Iu est CBZ-Val-Phe(H).
